# EUROPÄISCHE PATENTSCHRIFT

(11) **EP 3 380 026 B1**
(45) Veröffentlichungstag und Bekanntmachung des Hinweises auf die Patenterteilung: **14.12.2022**
(21) Anmeldenummer: 16795299.3
(22) Anmeldetag: 10.11.2016
(51) Int. Cl.: A61B 17/70

(54) **HANDHABUNGSINSTRUMENT FÜR EINE KNOCHENSCHRAUBE**
HANDLING INSTRUMENT FOR A BONE SCREW
INSTRUMENT DE MANIPULATION POUR UNE VIS DE FIXATION OSSEUSE

(30) Priorität: 26.11.2015 DE 102015223479
(43) Veröffentlichungstag der Anmeldung: 03.10.2018
(73) Patentinhaber: Silony Medical International AG, 8500 Frauenfeld (CH)
(72) Erfinder: HEUER, Frank, 70794 Filderstadt (DE)
(74) Vertreter: DREISS Patentanwälte PartG mbB
(86) Internationale Anmeldenummer: PCT/EP2016/077289
(87) Internationale Veröffentlichungsnummer: WO 2017/089141

(56) Entgegenhaltungen:
- US-A1- 2006 200 132
- US-A1- 2009 163 963
- US-A1- 2009 234 395
- US-A1- 2013 110 179

## Beschreibung

Die Erfindung betrifft ein Handhabungsinstrument für eine Knochenschraube, insbesondere eine Pedikelschraube, wobei die Knochenschraube einen Gewindeschaft und einen Kopf, insbesondere einen Gabelkopf, aufweist und das Handhabungsinstrument mit der Knochenschraube lösbar, jedoch starr und drehfest verbindbar ist, um den Gewindeschaft der Knochenschraube mittels des Handhabungsinstruments in einen Knochen einschrauben zu können, mit einem in der Längsrichtung erstreckten außenliegenden wenigstens abschnittsweise rohr- oder hülsenförmig ausgebildeten Gehäuseteil mit einer Wandung und einem in dem außenliegenden Gehäuseteil angeordneten innenliegenden, insbesondere kanülierten Stabteil, wobei das innenliegende Stabteil in eine drehmomentaufnehmende Werkzeugansetzstelle der Knochenschraube eingreifen kann.

Wird im Folgenden von einer Längsrichtung gesprochen, ist damit die axiale Längsrichtung gemeint. Die Umfangsrichtung ist konzentrisch zur axialen Längsrichtung, die radiale Richtung senkrecht zur axialen Längsrichtung. Das Handhabungsinstrument weist ein distales und ein diesem in der Längsrichtung gegenüberliegendes proximales Ende auf, wobei das Handhabungsinstrument im Bereich des distalen Endes mit der Knochenschraube verbindbar ist. Distal bedeutet damit in Richtung des distalen Endes des Handhabungsinstruments und proximal in Richtung des proximalen Endes des Handhabungsinstruments.

Derartige Instrumente zum Einschrauben von Knochenschrauben in einen Knochen sind aus dem Stand der Technik, beispielsweise aus der US 8,100,916 B2, bekannt. Nachteilig bei den bekannten Handhabungsinstrumenten ist, dass das Drehmoment zum Einschrauben der Knochenschraube nur über das zentrale Stabteil und eine Werkzeugansetzstelle der Knochenschraube eingeleitet wird. Ferner greifen die bekannten Instrumente in ein Innengewinde am Kopf der Knochenschraube ein, um den Kopf der Knochenschraube während des Einschraubens gegenüber dem Gewindeschaft stabil zu halten. Das Innengewinde kann dadurch beschädigt werden.

Ein Handhabungsinstrument mit den Merkmalen des Oberbegriffs des Anspruchs 1 ist bekannt aus US 2009/0163963 A1. Dessen innenliegendes Stabteil greift aber nicht unmittelbar in eine drehmomentaufnehmende Werkzeugansetzstelle der Knochenschraube ein sondern ist drehfest mit einem am distalen Ende des rohr- oder hülsenförmig ausgebildeten Gehäuseteils angeordneten weiteren Drehantriebs-Hülsenteil gekoppelt. Dieses weitere Hülsenteil greift mit axialen stiftförmigen Vorsprüngen zur Drehmomentübertragung in das proximale Ende des Gewindeschafts der Knochenschraube. Wenn das innenliegende Stabteil in der axialen Längsrichtung in Richtung auf die Knochenschraube bewegt wird, so verdrängt es Arme des rohroder hülsenförmigen ausgebildeten Gehäuseteils nach radial außen, welche dann eine drehfeste Kopplung des rohr- oder hülsenförmigen ausgebildeten Gehäuseteils zum Gabelkopf der Knochenschraube bewirken. Zum Verstellen des Kopplungselements und damit des innenliegenden Stabteils in der axialen Längsrichtung ist eine äußere Schiebehülse vorgesehen, die aber nicht arretierbar ist.

Der vorliegenden Erfindung liegt die Aufgabe zugrunde, ein Handhabungsinstrument derart auszubilden, dass einerseits die Kinematik beim Verbinden des Handhabungsinstruments mit der Knochenschraube und andererseits beim Einschrauben der Knochenschraube in einen Knochen verbessert wird und das Instrument so für den Chirurgen komfortabler anwendbar ist.

Diese Aufgabe wird erfindungsgemäß durch ein Handhabungsinstrument mit den Merkmalen des Anspruchs 1 gelöst.

Das außenliegende Gehäuseteil und das innenliegende Stabteil des erfindungsgemäßen Handhabungsinstruments sind durch das Kopplungselement drehfest miteinander gekoppelt. Das Drehmoment zum Einschrauben der Knochenschraube wird hierdurch sowohl über das in eine Werkzeugansetzstelle der Knochenschraube eingreifende Stabteil als auch über das außenliegende Gehäuseteil übertragen. Mittels des Kopplungselements ist es möglich, das Stabteil relativ zu dem Gehäuseteil in axialer Längsrichtung zu verschieben und somit in der Längsrichtung zu belasten. Durch Verschieben des Stabteils bezüglich des Gehäuseteils in Längsrichtung nach distal, ist es möglich, Stabteil und Gehäuseteil gegenüber der Knochenschraube zu verspannen. Vorzugsweise sind Mittel vorgesehen, um das außenliegende Gehäuseteil mit der Knochenschraube zu verbinden.

Es ist denkbar, dass das Kopplungselement schwenkbar am Stabteil gehalten ist, und dadurch von radial innen in den Führungsschlitz des Gehäuseteils hinein schwenkbar ist. Gemäß einer vorteilhaften Ausgestaltung der Erfindung ist die Queröffnung des Stabteils eine Einstecköffnung, in die das Kopplungselement von außen in radialer Richtung durch den Führungsschlitz des außenliegenden Gehäuseteils einsteckbar ist. Das Stabteil ist derart in dem Gehäuseteil angeordnet, dass die Einstecköffnung des Stabteils hinter dem Führungsschlitz des Gehäuseteils positioniert ist. Dadurch ist das Kopplungselement von außen in radialer Richtung in die Einstecköffnung des innenliegenden im Gehäuseteil angeordneten Stabteils einsteckbar.

Nach einer weiteren vorteilhaften Ausgestaltung der Erfindung weist der Führungsschlitz des außenliegenden Gehäuseteils eine in der Längsrichtung variierende Schlitzbreite auf. Dadurch wird ein Einstecken des Kopplungselements erleichtert und die Führungsfunktion für das Kopplungselement gewährleistet.

Hierbei erweist es sich als vorteilhaft, wenn das Kopplungselement im eingesteckten Zustand nach radial außen verjüngt ist, und an mindestens einer bestimmten, vorzugsweise die größte Schlitzbreite aufweisenden Position, durch den Führungsschlitz des außenliegenden Gehäuseteils in die Einstecköffnung des innenliegenden Stabteils ein- oder aussteckbar ist. Der verjüngte radial äußere Endbereich des Kopplungselements ist vorzugsweise über die gesamte Erstreckung des Führungsschlitzes in axialer Längsrichtung darin gleitverschieblich. Aufgrund der Verjüngung nach radial außen und der variierenden Breite des Führungsschlitzes ist das Kopplungselement verliersicher in dem Gehäuseteil angeordnet.

Nach einem nicht beanspruchten Beispiel wird vorgeschlagen, dass eine Verriegelungshülse mit einer Einstecköffnung für das Kopplungselement konzentrisch zwischen dem außenliegenden Gehäuseteil und dem innenliegenden Stabteil anordenbar ist, und dass die Queröffnung im Stabteil eine in axialer Richtung erstreckte Langlochöffnung ist, so dass die Verriegelungshülse mittels des Kopplungselements relativ zu dem Stabteil und/oder dem Gehäuseteil in axialer Längsrichtung verschiebbar ist. Das Kopplungselement ist vorzugsweise von radial außen durch den Führungsschlitz des außenliegenden Gehäuseteils und durch die Einstecköffnung der Verriegelungshülse in die Langlochöffnung des Stabteils einsteckbar. Das Kopplungselement ist in der Langlochöffnung des innenliegenden Stabteils und in dem Führungsschlitz des außenliegenden Gehäuseteils in der axialen Längsrichtung gleitverschieblich. Dadurch ist die Verriegelungshülse relativ zu dem Stabteil und/oder dem Gehäuseteil in axialer Längsrichtung verschiebbar bis jeweils das quererstreckte Kopplungsteil am Ende des Langlochs des Stabteils anschlägt.

Erfindungsgemäß wird also vorgeschlagen, dass am außenliegenden Gehäuseteil ein Kniehebelmechanismus vorgesehen ist, wobei der Kniehebelmechanismus ein erstes und ein zweites Hebelelement umfasst und das erste Hebelelement mit dem zweiten Hebelelement über ein Kniegelenk gelenkig verbunden ist und das erste Hebelelement schwenkbar am äußeren Gehäuseteil abgestützt ist und das zweite Hebelelement am Kopplungselement abgestützt ist, und der Kniehebelmechanismus durch Betätigen eines Bedienelements von einem gebeugten Zustand in einen gestreckten Zustand und umgekehrt bewegbar ist, so dass durch Betätigen des Kniehebelmechanismus das Kopplungselement in der Längsrichtung nach distal bzw. proximal relativ zum außenliegenden Gehäuseteil bewegbar ist. Es erweist sich als vorteilhaft, wenn das erste und/oder zweite Hebelelement halbschalenförmig ausgebildet ist und im gestreckten Zustand des Kniehebelmechanismus das außenliegende Gehäuseteil halbschalenförmig umgibt und vorzugsweise an diesem anliegt.

Das schwenkbar am außenliegenden Gehäuseteil abgestützte erste Hebelelement kann mittels des Bedienelements verschwenkt werden. Über das gelenkig mit diesem verbundene zweite Hebelelement wird die Bewegung auf das gleitverschieblich im Führungsschlitz des Gehäuseteils angeordnete Kopplungselement übertragen. Auf diese Weise wird eine Schwenkbewegung des Bedienelements in eine Linearbewegung des Kopplungselements in Längsrichtung nach distal bzw. proximal umgesetzt, und es ist möglich das Stabteil relativ zu dem Gehäuseteil in axialer Längsrichtung zu verschieben und zu belasten. Im gestreckten Zustand wird die Spannkraft des Kniehebelmechanismus über das Kopplungselement auf das Stabteil übertragen und Stabteil und Gehäuseteil können gegenüber der Knochenschraube verspannt werden.

Es erweist sich als vorteilhaft, dass das zweite Hebelelement mindestens eine Aufnahmeöffnung für das Kopplungselement aufweist. Vorteilhafterweise greift das Kopplungselement mit seinem radial äußeren durch den Führungsschlitz des außenliegenden Gehäuseteils herausragenden Ende in die Aufnahmeöffnung des Hebelelements ein.

Weiter erfindungsgemäß ist vorgesehen, dass der Kniehebelmechanismus ein elastisches Element umfasst, das von dem ersten oder zweiten Hebelelement gebildet wird. Vorteilhafterweise wird mit dem elastischen Element über das Kopplungselement beim Schließen des Kniehebelmechanismus, d.h. wenn der Kniehebelmechanismus in den gestreckten Zustand bewegt wird, eine Vorspannung auf das Stabteil übertragen. Im gestreckten Zustand des Kniehebelmechanismus wird das elastische Element insbesondere zwischen 0,1 mm und 5 mm, vorzugsweise zwischen 0,5 mm und 2 mm gestaucht. Vorzugsweise kann mit dem Kniehebelmechanismus eine Kraft von 100 N (Newton) bis 10 kN, vorzugsweise von 250 N bis 5 kN erzeugt werden. Beim Lösen des Kniehebelmechanismus, d.h. wenn der der Kniehebelmechanismus in den gebeugten Zustand bewegt wird, bleibt die Vorspannung zunächst erhalten und baut erst ab, wenn der Kniehebelmechanismus hinreichend geöffnet ist.

Vorteilhafterweise weist das erste oder zweite Hebelelement in einer Seitenansicht trapez-, dreieck- oder bogenförmig, insbesondere C-bogenförmig, angeordnete Streben auf, die einenends starr miteinander verbunden sind und anderenends nicht oder jedenfalls nicht starr miteinander verbunden sind. Vorzugsweise wird durch Zusammendrücken der freien oder jedenfalls nicht starr verbundenen Enden der Streben eine Vorspannkraft aufgebaut.

Nach einer weiteren vorteilhaften Ausgestaltung der Erfindung weist das außenliegende Gehäuseteil in einem distalen Endbereich zwei ungefähr halbschalenförmige in Längsrichtung diametral zueinander angeordnete Abschnitte auf, die durch einen dazwischen ausgebildeten, in der Längsrichtung erstreckten Spalt begrenzt sind und geringfügig quer zur Längsrichtung gegeneinander auslenkbar sind, so dass sie in eine Hintergriffstellung am Kopf der Knochenschraube gelangen, insbesondere einrasten oder einschnappen können und dabei zumindest einen Teil des Kopfs der Knochenschraube zwischen sich aufnehmen. Die halbschalenförmigen Abschnitte hintergreifen vorteilhafterweise zumindest einen Teil der Knochenschraube in der axialen Längsrichtung und in der Umfangsrichtung. Nach einer weiteren vorteilhaften Ausgestaltung der Erfindung weist das innenliegende Stabteil mindestens einen Querstift auf, der von radial innen in den Spalt zwischen den zwei ungefähr halbschalenförmigen Abschnitten hineinragt, so dass durch Bewegen des innenliegenden Stabteils in Längsrichtung nach proximal relativ zum außenliegenden Gehäuseteil, der Querstift gegen den Spalt begrenzende Schrägen anläuft und so die halbschalenförmigen Abschnitte gegeneinander aufspreizt.

Auf diese Weise ist es möglich, den Querstift, welcher in den Spalt zwischen den zwei halbschalenförmigen Abschnitten hineinragt, mittels des Kopplungselements in der Längsrichtung zu verstellen und durch Verschieben des Kopplungselements in axialer Längsrichtung nach proximal die halbschalenförmigen Abschnitte gegeneinander aufzuweiten. In diesem aufgespreizten Zustand der halbschalenförmigen Abschnitte ist der Kopf der Knochenschraube zwischen die beiden Abschnitte einbringbar. Beim Verschieben des Kopplungselements nach distal wird der Querstift von den Schrägen wegbewegt, und die halbschalenförmigen Abschnitte gelangen, beziehungsweise federn, wieder in ihre Ausgangsposition zurück und hintergreifen einen Teil des Kopfs der Knochenschraube.

Es erweist sich als vorteilhaft, dass durch Eingreifen des Stabteils in die Werkzeugansetzstelle an der Knochenschraube und durch einen Hintergriff in Umfangsrichtung oder in axialer Längsrichtung des außenliegenden Gehäuseteils am Kopf der Knochenschraube eine Verspannung des Gehäuseteils und des Stabteils gegeneinander und gegenüber der Knochenschraube bewirkbar ist. Wenn sich die beiden halbschalenförmigen Abschnitte in einer Hintergriffstellung mit dem Kopf der Knochenschraube befinden, und das in die Werkzeugansetzstelle an der Knochenschraube eingreifende Stabteil in Längsrichtung weiter nach distal verschoben wird, wird dadurch das Stabteil und das Gehäuseteil gegeneinander verspannt. Auf diese Weise wird auch der Gewindeschaft der Knochenschraube gegenüber dem Kopf der Knochenschraube verspannt, und das Drehmoment zum Einschrauben der Knochenschraube kann sowohl über den Kopf der Knochenschraube als auch über die Werkzeugansetzstelle übertragen werden.

Nach einer weiteren vorteilhaften Ausgestaltung der Erfindung ist ein Verriegelungselement vorgesehen, das die halbschalenförmigen Abschnitte des außenliegenden Gehäuseteils zumindest teilweise derart übergreift, dass ein Aufspreizen der halbschalenförmigen Abschnitte zumindest teilweise verhindert wird. Vorzugsweise übergreift das Verriegelungselement die halbschalenförmigen Abschnitte dann, wenn diese sich in einer Hintergriffstellung mit der Knochenschraube befinden und ein Lösen des Hintergriffs verhindert werden soll.

Das insbesondere ringförmige Verriegelungselement ist gemäß einer bevorzugten Ausführungsform am Querstift des Stabteils gehalten. Auf diese Weise kann das Verriegelungselement durch Verschieben des Stabteils mittels des Kopplungselements in axialer Richtung verschoben werden. Durch ein Verschieben des Kopplungselementes in axialer Richtung nach distal und ein damit einhergehendes Verschieben des Querstifts und des Verriegelungselements, gelangen also die halbschalenförmigen Abschnitte aus einer aufgeweiteten Stellung wieder in ihren Grundzustand zurück und das Verriegelungselement verriegelt die Abschnitte gegen ungewolltes Aufspreizen.

Nach einer weiteren vorteilhaften Ausgestaltung des erfindungsgemäßen Handhabungsinstruments ist in dem außenliegenden Gehäuseteil wenigstens eine radiale Ausnehmung ausgebildet, durch welche hindurch eine Markierung an einer Außenseite des innenliegenden Stabteils visuell wahrnehmbar ist, wenn sich das innenliegende Stabteil in einer bestimmten Position in Längsrichtung relativ zu dem außenliegenden Gehäuseteil befindet. Dies gestattet die gewünschte korrekte Positionierung des innenliegenden Stabteils in Längsrichtung relativ zu dem außenliegenden Gehäuseteil.

Nach einer weiteren vorteilhaften Ausgestaltung der Erfindung ist das innenliegenden Stabteil und vorzugsweise das Kopplungselement in axialer Längsrichtung kanüliert ausgebildet, sodass ein Führungsdraht darin hindurch geführt werden kann. Beispielsweise kann so ein Führungsdraht durch das Handhabungsinstrument und die Knochenschraube hindurch geführt werden, der an einer bestimmten Position am Knochen vorab fixiert ist, damit die Knochenschraube positioniert und an der richtigen Stelle am Knochen eingeschraubt werden kann.

Nach einer weiteren vorteilhaften Ausgestaltung des erfindungsgemäßen Handhabungsinstruments weist das außenliegende Gehäuseteil am proximalen Ende eine oder mehrere Werkzeugansetzstellen auf, so dass ein passendes Werkzeug so angesetzt werden kann, dass damit das Handhabungsinstrument um die Längsrichtung gedreht werden kann, um den Gewindeschaft der Knochenschraube in einen Knochen ein- bzw. auszuschrauben.

Weitere Vorteile, Merkmale und Einzelheiten der Erfindung ergeben sich aus den Unteransprüchen sowie der zeichnerischen Darstellung und nachfolgenden Beschreibung der in den Figuren dargestellten bevorzugten Ausführungsformen.

In der Zeichnung zeigt:
- Figur 1: eine Darstellung eines Handhabungsinstruments in einer ersten nicht beanspruchten bevorzugten Ausführungsform;
- Figuren 2a und 2b: das Handhabungsinstrument gemäß Fig. 1 in verschiedenen Handhabungsschritten;
- Figur 3a: das Handhabungsinstrument gemäß Fig. 1 in einer Seitenansicht;
- Figur 3b: das Handhabungsinstrument gemäß Fig. 1 in einer Draufsicht;
- Figur 3c: das Handhabungsinstrument gemäß Fig. 1 in einer Schnittansicht gemäß A-A aus Fig. 3b;
- Figur 3d: ein Teil das Handhabungsinstrument gemäß Fig. 1;
- Figur 3e: ein weiterer Teil das Handhabungsinstrument gemäß Fig. 1;
- Figur 4: eine Darstellung einesHandhabungsinstruments in einer zweiten nicht beanspruchten bevorzugten Ausführungsform;
- Figur 5: eine Darstellung einesHandhabungsinstruments in einer dritten nicht beanspruchten bevorzugten Ausführungsform;
- Figuren 6a bis 6d: das Handhabungsinstruments gemäß Fig. 5 in verschiedenen Handhabungsschritten;
- Figur 7: eine Darstellung des erfindungsgemäßen Handhabungsinstruments in einer vierten bevorzugten Ausführungsform, und
- Figuren 8a und 8b: das Handhabungsinstrument gemäß Fig. 7 in verschiedenen Handhabungsschritten.

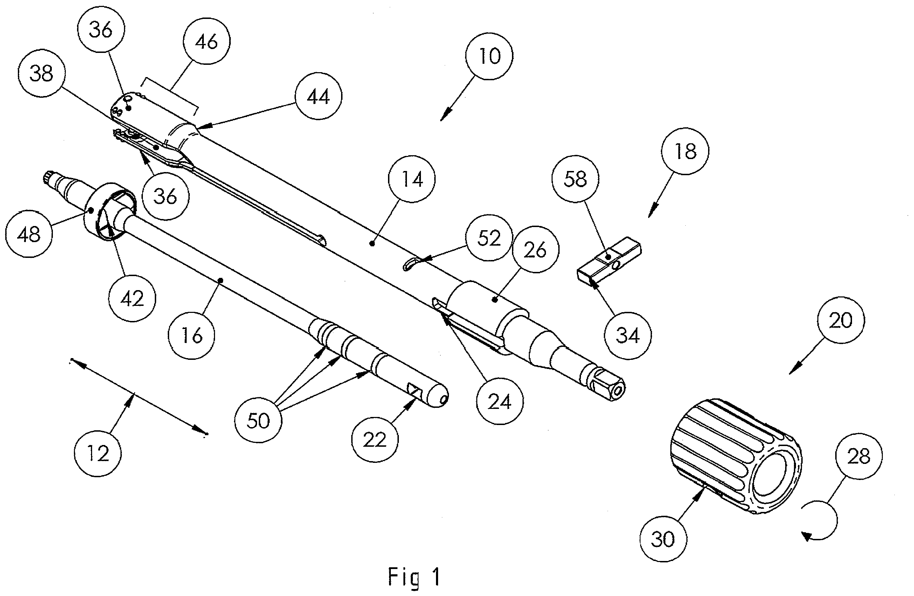

Die Figuren zeigen ein insgesamt mit dem Bezugszeichen 10 bezeichnetes Handhabungsinstrument. Gemäß einer ersten nicht beanspruchten Ausführungsform nach Fig. 1 umfasst das Handhabungsinstrument 10 ein in der Längsrichtung 12 erstrecktes Gehäuseteil 14 und ein in der Längsrichtung 12 erstrecktes Stabteil 16 sowie ein zwischen diesen quererstrecktes Kopplungselement 18 und eine Gewindehülse 20.

Das Gehäuseteil 14 ist zumindest abschnittsweise ungefähr rohr- oder hülsenförmig und so ausgebildet, dass das Stabteil 16 in das außenliegende Gehäuseteil 14 in der Längsrichtung 12 eingeführt werden kann.

Damit das Stabteil 16 in dem Gehäuseteil 14 drehfest angeordnet werden kann, weist das Stabteil 16 eine Queröffnung 22 und das Gehäuseteil 14 einen Führungsschlitz 24 auf. In der beispielhaft dargestellten Ausführungsform ist das Kopplungselement 18 von radial außen durch den Führungsschlitz 24 des Gehäuseteils 14 in die Queröffnung 22 des im Gehäuseteil 14 angeordneten Stabteils 16 einsteckbar. Das Kopplungselement 18 ragt im eingesteckten Zustand in den Führungsschlitz 24 hinein und ist darin in Längsrichtung gleitverschieblich angeordnet. Auf diese Weise ist das Stabteil 16 mittels des Kopplungselements 18 im Gehäuseteil 14 drehfest jedoch in der Längsrichtung 12 relativ zum Gehäuseteil 14 verschieblich. Durch Verschieben des Kopplungselements 18 in der Längsrichtung 12 ist also das Stabteil 16 relativ zum Gehäuseteil 14 verschiebbar. Es ist denkbar, dass das Stabteil 16 gegenüber dem Gehäuseteil 14, insbesondere mit einer Feder (hier nicht dargestellt), vorspannbar ist.

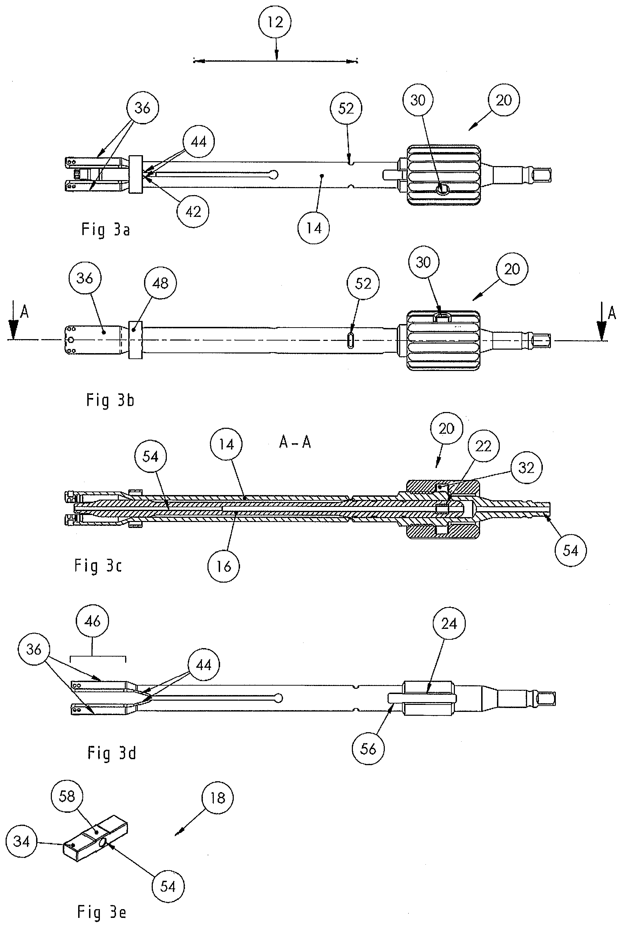

Das Gehäuseteil 14 umfasst ein Außengewinde 26. Das Außengewinde 26 erstreckt sich in einer Umfangsrichtung 28 und ist im Bereich des Führungsschlitzes 24 des Gehäuseteils 14 unterbrochen. Die Gewindehülse 20 ist auf das Außengewinde 26 aufschraubbar. Die Gewindehülse 20 weist eine Einstecköffnung 30 für das Kopplungselement 18 und eine Innenumfangsnut 32 (siehe Fig. 3b) auf. Das Kopplungselement 18 ist von radial außen durch die Einstecköffnung 30 der auf das außenliegende Gehäuseteil 14 aufgeschraubten Gewindehülse 20 und durch den Führungsschlitz 24 des Gehäuseteils 14 in die Queröffnung 22 des Stabteils 16 einsteckbar. Ein radial äußeres Ende 34 des Kopplungselements 18 ragt im eingesteckten Zustand beidseits nach radial außen durch den Führungsschlitz 24 des Gehäuseteils 14 heraus und greift in die Innenumfangsnut 32 der Gewindehülse 20 ein. Beim Auf- bzw. Abschrauben der Gewindehülse 20 führt diese also eine Relativbewegung in Umfangsrichtung 28 gegenüber dem Kopplungselement 18 und den mittels des Kopplungselements 18 drehfest zueinander angeordneten Stab- und Gehäuseteil 14 aus. Das Kopplungselement 18 wird beim Auf- bzw. Abschrauben der Gewindehülse 20 in Längsrichtung 12 relativ zum außenliegenden Gehäuseteil 14 verschoben. Auf diese Weise ist das Stabteil 16 in Längsrichtung 12 relativ zum Gehäuseteil 14 mittels der Gewindehülse 20 verschiebbar.

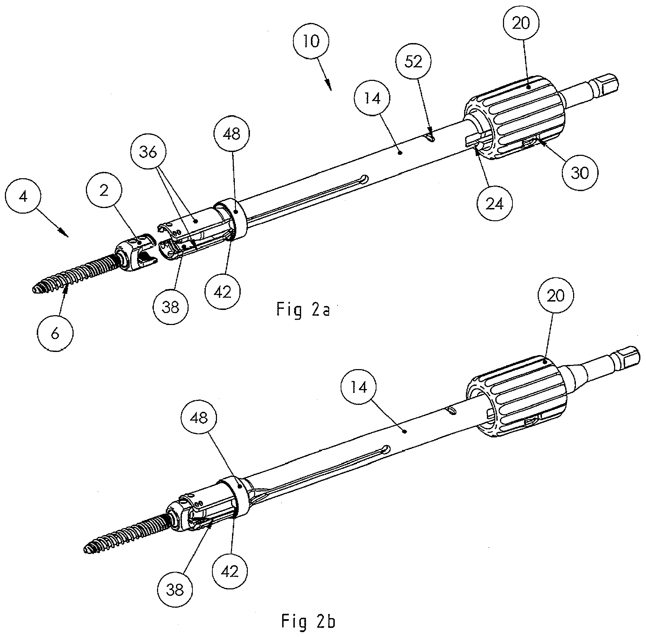

Das Gehäuseteil 14 umfasst zwei ungefähr halbschalenförmige in Längsrichtung 12 diametral zueinander angeordnete Abschnitte 36, die durch einen dazwischen ausgebildeten, in der Längsrichtung erstreckten Spalt 38 begrenzt sind. Die halbschalenförmigen Abschnitte 36 sind quer zur Längsrichtung 12 geringfügig gegeneinander auslenkbar. Auf diese Weise können die beiden Abschnitte 36 in eine Hintergriffstellung am Kopf 2 der Knochenschraube 4 gelangen und dabei zumindest einen Teil des Kopfs 2 der Knochenschraube 4 zwischen sich aufnehmen (siehe Fig. 2b).

Das Stabteil 16 umfasst zwei Querstifte 42, die sich von radial innen von dem im außenliegenden Gehäuseteil 14 angeordneten Stabteil 16 nach radial außen in den die halbschalenförmigen Abschnitte 36 begrenzenden Spalt 38 erstrecken. Wird das Stabteils 16 relativ zum Gehäuseteil 14 in der Längsrichtung 12 nach proximal verschoben, laufen die Querstifte 42 gegen den Spalt 38 begrenzende Schrägen 44 an. Die beiden halbschalenförmigen Abschnitte 36 werden dadurch gegeneinander quer zur Längsrichtung 12 aufgespreizt (siehe Fig. 2a). In diesem aufgespreizten Zustand können die halbschalenförmigen Abschnitte 36 zumindest einen Teil des Kopfs 2 der Knochenschraube 4 zwischen sich aufnehmen, und durch Zurückfedern bzw. Zurückschnappen in eine Hintergriffstellung mit einem Teil des Knochenschraubenkopfs 2 gelangen. Es ist denkbar, dass in einem distalen Endbereich 46 der halbschalenförmigen Abschnitte 36 Mittel vorgesehen sind, die mit dazu komplementäre Mitteln am Knochenschraubenkopf 2 zusammenwirken, insbesondere darin eingreifen, einrasten oder einschnappen können, und dass durch die derartig zusammenwirkenden Mittel die halbschalenförmigen Abschnitte 36 in eine Hintergriffstellung mit dem Knochenschraubenkopf 2 in Längsrichtung 12 und vorzugsweise auch in Umfangsrichtung 28 gelangen.

Damit die halbschalenförmigen Abschnitte 36 in eine Hintergriffstellung mit dem Kopf 2 der Knochenschraube 4 gelangen können, wird das Stabteil 16 mittels des Kopplungselements 18 in Längsrichtung 12 relativ zum Gehäuseteil 14 nach distal verschoben. Dadurch entfernen sich die Querstifte 42 von den Schrägen 44 und die beiden halbschalenförmigen Abschnitten 36 federn wieder aufeinander zu. Das Stabteil 16 wird weiter in Längsrichtung 12 nach distal bewegt, bis es in eine Werkzeugansetzstelle am Gewindeschaft 6 der Knochenschraube 4 eingreift. Mittels der Hintergriffstellung des Gehäuseteils 14 mit dem Kopf 2 der Knochenschraube 4 und dem Eingreifen des Stabteils 16 in die Werkzeugansetzstelle am Gewindeschaft 6, sind das Gehäuseteil 14 und das Stabteil 16 gegeneinander und gegenüber der Knochenschraube 4 verspannbar. Ferner sind das Stabteil 16 und das Gehäuseteil 14 mittels des in den Führungsschlitz 24 und in die Queröffnung 22 eingesteckten Kopplungselements 18 und der in den Spalt 38 eingreifenden Querstifte 42 drehfest zueinander angeordnet. Auf diese Weise ist das Drehmoment zum Einschrauben der Knochenschraube 4 sowohl über das Gehäuseteil 14 als auch über das Stabteil 16 einleitbar.

An den Querstiften 42 des Stabteils 16 ist ein ringförmiges Verriegelungselement 48 angeordnet. Wird das Stabteil 16 in Längsrichtung 12 nach distal verschoben, wird das Verriegelungselement 48 über den distalen Endbereich 46 der hülsenförmigen Abschnitte 36 geschoben. Das Verriegelungselement 48 übergreift die Abschnitte 36 derart, dass diese gegen ein Aufspreizen verriegelt sind und die Hintergriffstellung nicht ungewollt gelöst werden kann.

Ferner weist das Stabteil 16 an einer Außenseite mehrere Markierungen 50 und das Gehäuseteil 14 eine Ausnehmung 52 auf, durch welche hindurch die Markierungen 50 ersichtlich sind. Dadurch ist es möglich, die relative Verschiebung in Längsrichtung 12 zwischen Stabteil 16 und Gehäuseteil 14 zu bestimmen.

Figur 3c zeigt eine Schnittansicht des erfindungsgemäßen Handhabungsinstrumentes 10 gemäß einem Schnitt an A-A (siehe Fig. 3b). Das Stabteil 16, das Gehäuseteil 14 und das Kopplungselement 18 (siehe Fig. 3e) weisen eine in der Längsrichtung 12 erstreckte Durchgangsöffnung 54 auf und sind dadurch in der Längsrichtung 12 kanüliert ausgebildet. In der Durchgangsöffnung 54 kann beispielsweise ein Führungsdraht durch das Handhabungsinstrument 10 und die Knochenschraube 4 hindurch geführt werden, der an einer bestimmten Position am Knochen vorab fixiert ist, damit die Knochenschraube 4 positioniert und an der richtigen Stelle am Knochen eingeschraubt werden kann.

Figur 3d zeigt eine Seitenansicht auf das Gehäuseteil 14. Der Führungsschlitz 24 weist eine in der Längsrichtung 12 variierende Breite auf. Die Schlitzbreite variiert insbesondere zwischen 1,5 mm und 20 mm, insbesondere zwischen 2 mm und 10 mm. In der beispielhaft dargestellten Ausführungsform nimmt die Breite des Führungsschlitzes 24 ausgehend von einem distalen, die größte Breite aufweisenden Ende 56 in Längsrichtung 12 nach proximal ab. Das Kopplungselement 18 verjüngt sich nach radial außen und der Querschnitt des Kopplungselement 18 nimmt ausgehend von dem radial äußeren Ende 34 nach radial innen zu. Aufgrund der Querschnittszunahme 58 ist das Kopplungselement 18 nur an einer Position, in dem dargestellten Ausführungsbeispiel am distalen Ende 56 des Führungsschlitzes 24, durch diesen hindurch steckbar. Das verjüngte radial äußere Ende 34 des Kopplungselements 18 ist über die gesamte Erstreckung des Führungsschlitzes 24 in Längsrichtung 12 darin gleitverschieblich. Das Kopplungselement 18 ist durch die Querschnittszunahme 58 ausfallsicher in dem Gehäuseteil 14 angeordnet.

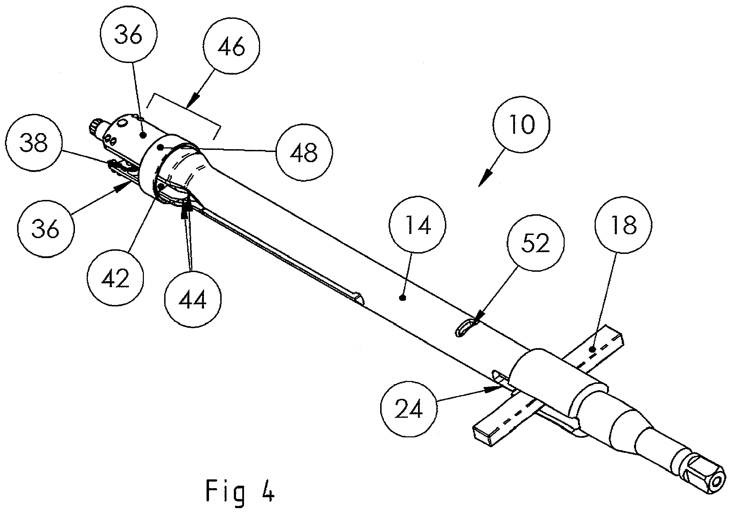

Figur 4 zeigt das Handhabungsinstrument 10 gemäß einer zweiten bevorzugten Ausführungsform. Das Stabteil 16 ist mittels des Kopplungselements 18 drehfest jedoch in Längsrichtung 12 relativ zum Gehäuseteil 14 gleitverschieblich in diesem angeordnet. Vorzugsweise ist das Stabteil 16 mittels einer Feder gegenüber dem Gehäuseteil 14 nach distal vorgespannt. Wird das Kopplungselement beispielsweise manuell in Längsrichtung 12 nach proximal zurückgezogen, wird die Verriegelung der halbschalenförmigen Abschnitte 36 gelöst und die halbschalenförmigen Abschnitte 36 mittels der Querstifte 42, welche gegen den Spalt 38 begrenzende Schrägen 44 anlaufen, gegeneinander aufgepreizt. Der Kopf 2 der Knochenschraube 4 oder zumindest ein Teil davon kann nun zwischen den zwei halbschalenförmigen Abschnitten 36 angeordnet werden. Wird das Kopplungselement 18 nicht mehr nach proximal entgegen der Vorspannkraft gezogen, drückt die in Längsrichtung 12 nach distal wirkende Vorspannkraft das Stabteil 16 relativ zum Gehäuseteil 14 in der Längsrichtung 12 nach distal. Das Stabteil 16 greift schließlich in eine Werkzeugansetzstelle der Knochenschraube 4 ein und das Gehäuseteil 14 hintergreift mittels der halbschalenförmigen Abschnitte 36 einen Teil des Kopfs 2 der Knochenschraube 4. Über die auf das Stabteil 16 wirkende Vorspannkraft und die Hintergriffstellung der Gehäuseteils 14 mit der Knochenschraube 4, sind das Stabteil 16 und das Gehäuseteil 14 gegeneinander und gegenüber der Knochenschraube 4 verspannt.

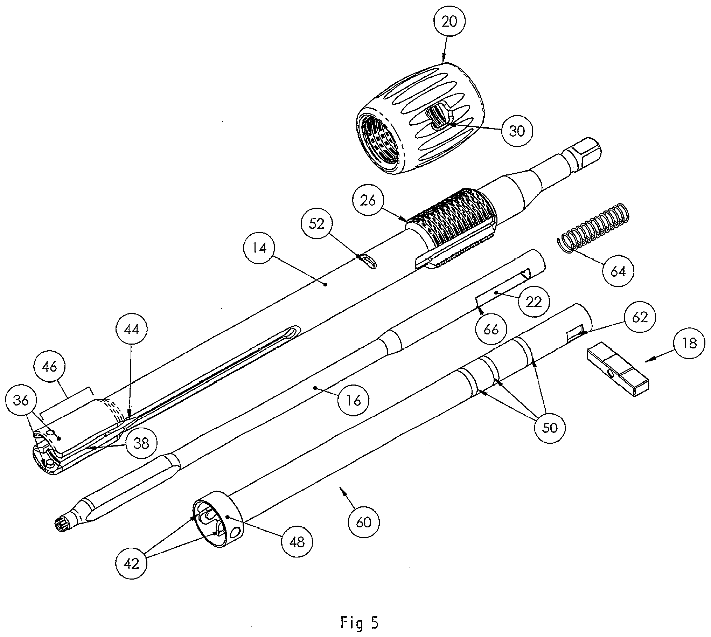

Figur 5 zeigt das Handhabungsinstrument 10 gemäß einer dritten bevorzugten Ausführungsform. Das Handhabungsinstrument 10 umfasst eine zusätzliche in der Längsrichtung 12 erstreckte Verriegelungshülse 60, die in der Längsrichtung 12 in das Gehäuseteil 14 einführbar ist. Die Verriegelungshülse 60 ist also konzentrisch zwischen Stabteil 16 und Gehäuseteil 14 anordenbar. Die Verriegelungshülse 60 weist ebenfalls eine Einstecköffnung 62 für das Kopplungselement 18 auf. Auf diese Weise ist die Verriegelungshülse 60 mittels des Kopplungselements 18 im Gehäuseteil 14 drehfest jedoch in der Längsrichtung 12 relativ zum Gehäuseteil 14 verschieblich anordenbar. Durch Verschieben des Kopplungselements 18 in der Längsrichtung 12 ist also die Verriegelungshülse 60 relativ zum Gehäuseteil 14 verschiebbar. Die Querstifte 42 zum Aufspreizen der halbschalenförmigen Abschnitte 36 und das Verriegelungselement 48 zum Sichern der Hintergriffstellung des Gehäuseteils 14 mit dem Kopf 2 der Knochenschraube 4 sind in diesem Ausführungsbeispiel an der Verriegelungshülse 60 angeordnet. Das Aufspreizen und Verriegeln der halbschalenförmigen Abschnitte 36 erfolgt auch hier durch Verschieben des Kopplungselements 18 und ein damit einhergehendes Verschieben der Verriegelungshülse 60 mittels Auf- und Abschrauben der Gewindehülse 20.

Die Queröffnung 22 des Stabteils 16 zum Einstecken des Kopplungselements 18 ist hier aber als Langlochöffnung mit einer Erstreckung in Längsrichtung 12 ausgebildet. Auf diese Weise ist die Verriegelungshülse 60 in der Längsrichtung 12 relativ zum Stabteil 16 verschiebbar. Das Stabteil 16 ist mittels einer Feder 64 gegenüber dem Gehäuseteil 14 nach distal vorspannbar.

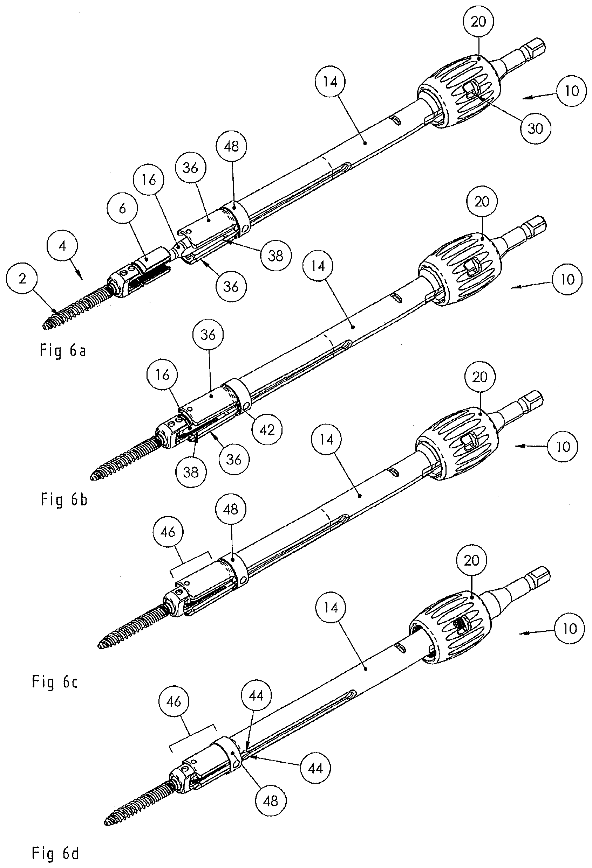

Die Figuren 6a bis 6d zeigen das Handhabungsinstrument 10 in verschiedenen Handhabungsschritten. In Figur 6a ist die Verriegelungshülse 60 mittels der Gewindehülse 20 relativ zum Gehäuseteil 14 und zum Stabteil 16 in Längsrichtung 12 nach proximal verschoben. Die an der Verriegelungshülse 60 angeordneten Querstifte 42 laufen gegen Schrägen 44, welche den Spalt 38 begrenzen, an. Hierdurch werden die halbschalenförmigen Abschnitte 36 quer zur Längsrichtung 12 gegeneinander aufgeweitet. Das Stabteil 16 ist mittels der Feder 64 vorgespannt und wird über die Vorspannkraft relativ zum Gehäuseteil 14 nach distal gedrückt. Das Stabteil 16 greift in die Werkzeugansetzstelle am Gewindeschaft 6 der Knochenschraube 4 ein (Fig. 6b). Damit der Kopf 2 der Knochenschraube 4 zwischen den halbschalenförmigen Abschnitten 36 angeordnet und mit diesen in eine Hintergriffstellung gelangen kann, wird das Stabteil 16 mit der Knochenschraube 4 in der Längsrichtung 12 nach proximal entgegen der Vorspannkraft belastet (Fig. 6c). Anschließend wird das Kopplungselement 18 und damit einhergehend die Verriegelungshülse 60 mittels der Gewindehülse 20 nach distal bewegt. Die halbschalenförmigen Abschnitte 36 federn wieder zurück und gelangen so in eine Hintergriffstellung mit der Knochenschraube 4. Das ringförmige Verriegelungselement 48 übergreift dann die halbschalenförmigen Abschnitte 36. Das Kopplungselement 18 wird weiter nach distal verschoben, bis es gegen ein distales Ende 66 der Langlochöffnung 22 anliegt und somit das Stabteil 16 über die nach distal wirkende Federkraft hinaus belastet. Auf diese Weise ist das Stabteil 16 gegenüber dem in einer Hintergriffstellung mit dem Kopf 2 der Knochenschraube 4 befindlichen Gehäuseteil 14 und gegenüber der Knochenschraube 4 verspannbar. Das Drehmoment zum Einschrauben der Knochenschraube 4 kann dann über den Kopf 2 und die Werkzeugansetzstelle der Knochenschraube 4 eingeleitet werden.

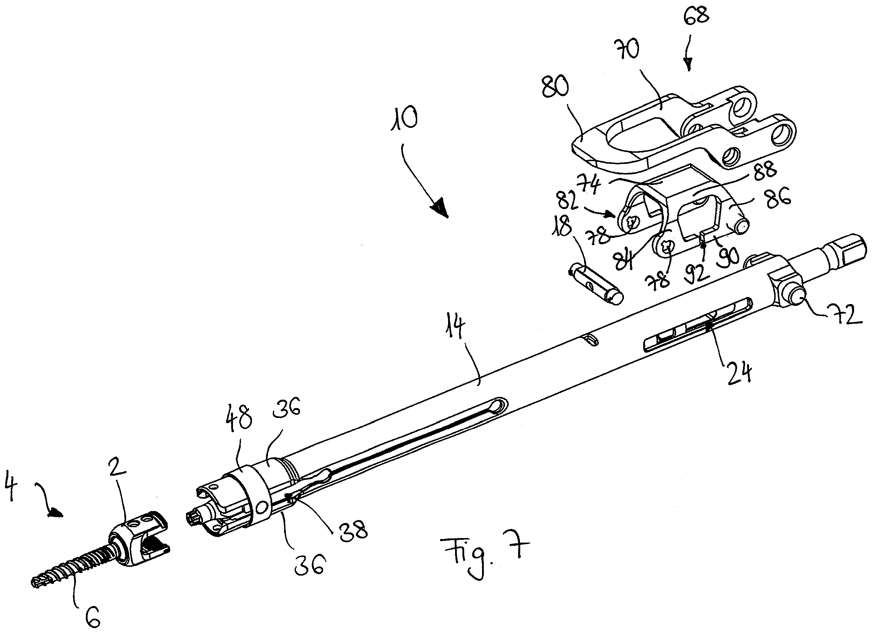

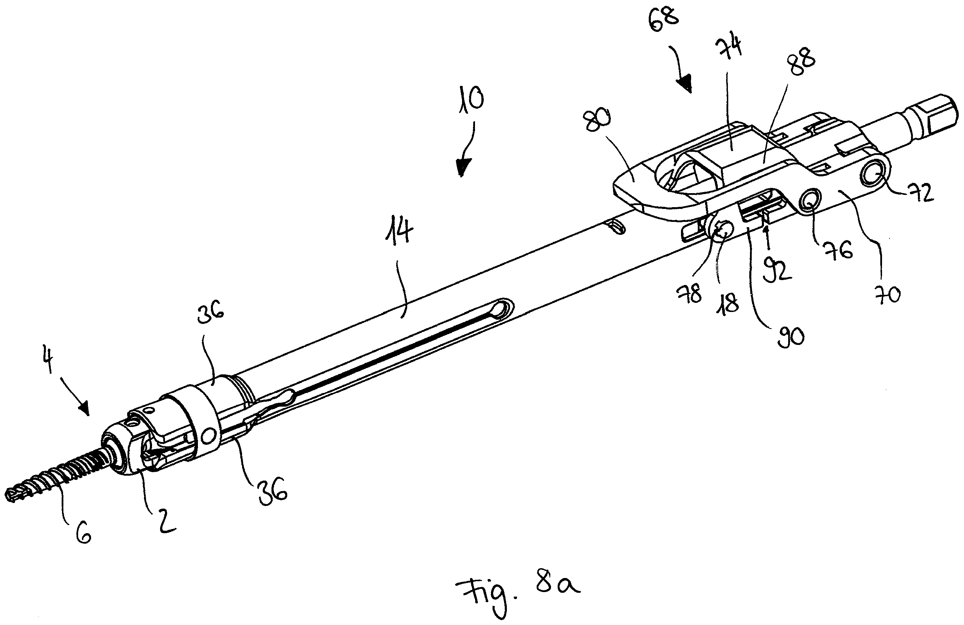

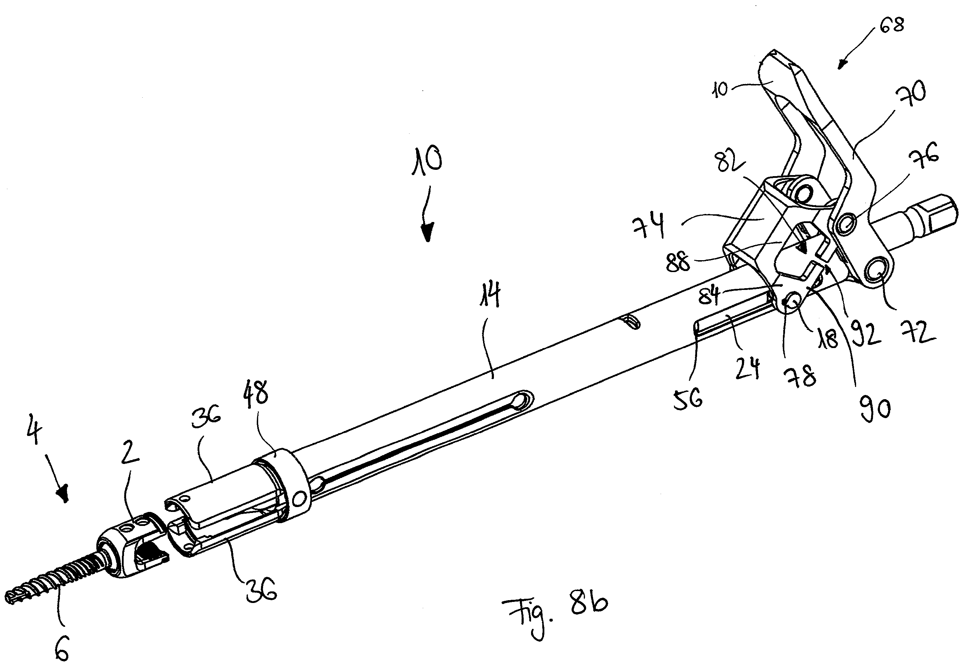

Figur 7 zeigt das Handhabungsinstrument 10 gemäß einer vierten bevorzugten erfindungsgemäßen Ausführungsform. Das Stabteil 16 ist mittels des Kopplungselements 18 drehfest jedoch in Längsrichtung 12 relativ zum Gehäuseteil 14 gleitverschieblich in diesem angeordnet. Zum Verschieben des Kopplungselements 18 umfasst das Handhabungsinstrument 10 einen Kniehebelmechanismus 68. Der Kniehebelmechanismus umfasst ein erstes Hebelelement 70, welches um eine Schwenkachse 72 schwenkbar am Gehäuseteil 14 abgestützt ist. Ein zweites Hebelelement 74 ist mit einem Kniegelenk 76 gelenkig mit dem ersten Hebelelement 70 verbunden (Fig. 8a und 8b). Das zweite Hebelelement 74 weist zwei Aufnahmeöffnungen 78 für das Kopplungselement 18 auf. Ist das Kopplungselement 18 in dem Führungsschlitz 24 des Gehäuseteils 14 angeordnet, greift das Kopplungselement 18 mit seinen radial äußeren durch den Führungsschlitz 24 des außenliegenden Gehäuseteils 14 herausragenden Enden in die Aufnahmeöffnungen 78 des zweiten Hebelelements 74 ein.

Der Kniehebelmechanismus 68 kann durch Betätigen eines Bedienelements 80 von einem gebeugten Zustand (Fig. 8b) in einen gestreckten Zustand (Fig. 8a) und umgekehrt bewegt werden. Das schwenkbar am außenliegenden Gehäuseteil 14 abgestützte erste Hebelelement 70 wird dabei mittels des Bedienelements 80 um die Schwenkachse 72 verschwenkt. Diese Schwenkbewegung wird über das Kniegelenk 76 auf das zweite Hebelelement 74 und damit auf das Kopplungselement 18 übertragen, wobei das Kopplungselement 18 eine Linearbewegung in Längsrichtung nach distal bzw. proximal ausführt.

In der dargestellten Ausführungsform ist das Kopplungselement 18 von radial außen durch den Führungsschlitz 24 des Gehäuseteils 14 in die Queröffnung 22 des im Gehäuseteil 14 angeordneten Stabteils 16 eingesteckt. Wird nun das Kopplungselement 18 mittels des Kniehebelmechanismus 68 in dem Führungsschlitz 24 bewegt, wird damit das Stabteil 16 relativ zu dem Gehäuseteil 14 in axialer Längsrichtung 12 verschoben und belastet.

Im gestreckten Zustand des Kniehebelmechanismus 68 wird die Spannkraft des Kniehebelmechanismus 68 über das Kopplungselement 18 auf das Stabteil 16 übertragen und Stabteil 16 und Gehäuseteil 14 können gegenüber der Knochenschraube 4 verspannt werden.

Der Kniehebelmechanismus 68 umfasst ferner ein elastisches Element 82. Gemäß der gezeigten Ausführungsform ist das elastische Element 82 am zweiten Hebelelement 74 ausgebildet. Das zweite Hebelelement 74 umfasst auf jeder Seite in einer Seitenansicht zwei trapezförmig angeordnete Streben 84, 86, die einenends, d.h. an einer oberen Grundseite 88, starr miteinander verbunden sind und anderenends, d.h. an einer unteren Grundseite 90, nicht miteinander verbunden sind, sodass an der unteren Grundseite 90 ein Spalt 92 ausgebildet wird.

Die erste Strebe 84 umfasst die Aufnahmeöffnung 78 für das Kopplungselement 18, sodass sich das zweite Hebelelement 74 über die erste Strebe 84 am Kopplungselement abstützt. Über die zweite Strebe 86 ist das zweite Hebelelement 74 über das Kniegelenk 76 mit dem ersten Hebelelement 70 verbunden. Beim Schließen des Kniehebelmechanismus wird nun die Strebe 86 in Richtung der Strebe 84 gedrückt und so eine Vorspannkraft aufgebaut. Diese Vorspannkraft wird über das Kopplungselement 18 auf das Stabteil 16 übertragen. Im gestreckten Zustand des Kniehebelmechanismus 86 wird das elastische Element 82 insbesondere zwischen 0,1 mm und 5 mm, vorzugsweise zwischen 0,5 mm und 2 mm gestaucht. Vorzugsweise kann mit dem Kniehebelmechanismus 68 eine Kraft von 100 N (Newton) bis 10 kN, vorzugsweise von 250 N bis 5 kN erzeugt werden. Beim Lösen des Kniehebelmechanismus 68, d.h. wenn der Kniehebelmechanismus 68 vom gestreckten in den gebeugten Zustand bewegt wird, bleibt die Vorspannung zunächst erhalten und baut erst ab, wenn der Kniehebelmechanismus 68 hinreichend geöffnet ist. Der Spalt 92 verhindert eine Überbeanspruchung des elastischen Elements 82, indem der Spalt 92 auch als Anschlag funktioniert.

In einem distalen Bereich des Handhabungsinstrument 10 umfasst das Stabteil 16 zwei Querstifte 42, die sich von radial innen von dem im außenliegenden Gehäuseteil 14 angeordneten Stabteil 16 nach radial außen in einen die halbschalenförmigen Abschnitte 36 begrenzenden Spalt 38 erstrecken. Wird das Stabteils 16 relativ zum Gehäuseteil 14 in der Längsrichtung 12 nach proximal verschoben, laufen die Querstifte 42 gegen den Spalt 38 begrenzende Schrägen 44 an. Die beiden halbschalenförmigen Abschnitte 36 werden dadurch gegeneinander quer zur Längsrichtung 12 aufgespreizt (siehe Fig. 8b). In diesem aufgespreizten Zustand können die halbschalenförmigen Abschnitte 36 zumindest einen Teil des Kopfs 2 der Knochenschraube 4 zwischen sich aufnehmen, und durch Zurückfedern bzw. Zurückschnappen in eine Hintergriffstellung mit einem Teil des Knochenschraubenkopfs 2 gelangen.

Wird das Kopplungselement 18 in Längsrichtung 12 durch Öffnen des Kniehebelmechanismus 68 nach proximal zurückgezogen, wird die Verriegelung der halbschalenförmigen Abschnitte 36 gelöst und die halbschalenförmigen Abschnitte 36 mittels der Querstifte 42, welche gegen den Spalt 38 begrenzende Schrägen 44 anlaufen, gegeneinander aufgepreizt. Der Kopf 2 der Knochenschraube 4 oder zumindest ein Teil davon kann nun zwischen den zwei halbschalenförmigen Abschnitten 36 angeordnet werden. Wird das Kopplungselement 18 durch Schließen des Kniehebelmechanismus nach distal bewegt, drückt die in Längsrichtung 12 nach distal wirkende Spannkraft des Kniehebelmechanismus das Stabteil 16 relativ zum Gehäuseteil 14 in der Längsrichtung 12 nach distal. Das Stabteil 16 greift schließlich in eine Werkzeugansetzstelle der Knochenschraube 4 ein und das Gehäuseteil 14 hintergreift mittels der halbschalenförmigen Abschnitte 36 einen Teil des Kopfs 2 der Knochenschraube 4. Über die auf das Stabteil 16 wirkende Spannkraft und die Hintergriffstellung der Gehäuseteils 14 mit der Knochenschraube 4, sind das Stabteil 16 und das Gehäuseteil 14 gegeneinander und gegenüber der Knochenschraube 4 verspannt.

Die Figuren 8a und 8b zeigen das Handhabungsinstrument 10 aus Figur 7 in verschiedenen Handhabungsschritten. In Figur 8b befindet sich der Kniehebelmechanismus 68 in einem gebeugten Zustand, d.h. in einem geöffneten Zustand. Durch Betätigen des Bedienelements 80 wird die Schwenkbewegung des ersten Hebelelements 70 auf das zweite Hebelelement 74 und damit auf das Kopplungselement 18 übertragen. Das Kopplungselement 18 bewegt sich beim Schließen des Kniehebelmechanismus, d.h. wenn der Kniehebelmechanismus 68 von dem gebeugten in einen gestreckten Zustand gebracht wird, nach distal. Beim Öffnen des Kniehebelmechanismus, d.h. wenn der Kniehebelmechanismus 68 von dem gebeugten in einen gestreckten Zustand gebracht wird bewegt sich das Kopplungselement 18, nach proximal.

## Patentansprüche

1. Handhabungsinstrument (10) für eine Knochenschraube (4), insbesondere eine Pedikelschraube, wobei die Knochenschraube (4) einen Gewindeschaft (6) und einen Kopf (2), insbesondere einen Gabelkopf, aufweist und das Handhabungsinstrument (10) mit der Knochenschraube (4) lösbar, jedoch starr und drehfest verbindbar ist, um den Gewindeschaft (6) der Knochenschraube (4) mittels des Handhabungsinstruments (10) in einen Knochen einschrauben zu können, mit einem in einer axialen Längsrichtung (12) erstreckten außenliegenden wenigstens abschnittsweise rohr- oder hülsenförmig ausgebildeten Gehäuseteil (14) mit einer Wandung und einem in dem außenliegenden Gehäuseteil (14) angeordneten innenliegenden, insbesondere kanülierten, Stabteil (16), wobei das innenliegende Stabteil (16) in eine drehmomentaufnehmende Werkzeugansetzstelle der Knochenschraube (4) eingreifen kann, wobei das außenliegende Gehäuseteil (14) wenigstens einen in der axialen Längsrichtung (12) erstreckten, eine Wandung des Gehäuseteils durchgreifenden Führungsschlitz (24) aufweistund das innenliegende Stabteil (16) im Bereich des Führungsschlitzes (24) des außenliegenden Gehäuseteils (14) eine insbesondere in radialer Richtung erstreckte Queröffnung (22) aufweist, und dass ein Kopplungselement (18) in dieser Queröffnung (22) des im außenliegenden Gehäuseteil (14) angeordneten innenliegenden Stabteils (16) anordenbar ist, wobei das Kopplungselement (18) in den Führungsschlitz (24) des außenliegenden Gehäuseteils (14) hineinragt oder nach außen durch den Führungsschlitz (24) herausragt und in beiden Fällen in der Längsrichtung (12) in dem Führungsschlitz (24) gleitverschieblich ist, so dass das innenliegende Stabteil (16) und das außenliegende Gehäuseteil (14) in axialer Längsrichtung (12) relativ zueinander verschiebbar jedoch mittels des Kopplungselements (18) drehfest angeordnet sind und wobei das Stabteil (16) mittels des Kopplungselements (18) in der axialen Längsrichtung (12) belastbar ist, **dadurch gekennzeichnet, dass** am außenliegenden Gehäuseteil (14) ein Kniehebelmechanismus (68) vorgesehen ist, wobei der Kniehebelmechanismus (68) ein erstes und ein zweites Hebelelement (70, 74) umfasst und das erste Hebelelement (70) mit dem zweiten Hebelelement (74) über ein Kniegelenk (76) gelenkig verbunden ist und dass das erste Hebelelement (70) schwenkbar am äußeren Gehäuseteil (14) abgestützt ist und das zweite Hebelelement (74) am Kopplungselement (18) abgestützt ist, und der Kniehebelmechanismus (68) durch Betätigen eines Bedienelements (80) von einem gebeugten Zustand in einen gestreckten Zustand und umgekehrt bewegbar ist, so dass durch Betätigen des Kniehebelmechanismus (68) das Kopplungselement (18) in der Längsrichtung (12) nach distal bzw. proximal relativ zum außenliegenden Gehäuseteil (14) bewegbar ist, und dass der Kniehebelmechanismus (68) ein elastisches Element (82) umfasst, das von dem ersten oder zweiten Hebelelement (70, 74) gebildet wird.

2. Handhabungsinstrument (10) nach Anspruch 1, **dadurch gekennzeichnet, dass** die Queröffnung (22) des Stabteils (16) eine Einstecköffnung ist, in die das Kopplungselement (18) von außen in radialer Richtung durch den Führungsschlitz (24) des außenliegenden Gehäuseteils (14) einsteckbar ist.

3. Handhabungsinstrument (10) nach Anspruch 1 oder 2, **dadurch gekennzeichnet, dass** der Führungsschlitz (24) eine in der Längsrichtung (12) variierende Schlitzbreite aufweist.

4. Handhabungsinstrument (10) nach Anspruch 3, **dadurch gekennzeichnet, dass** das Kopplungselement (18) nach radial außen verjüngt ist, und das Kopplungselement (18) an mindestens einer bestimmten, vorzugsweise die größte Schlitzbreite aufweisenden Position, durch den Führungsschlitz (24) des außenliegenden Gehäuseteils (14) in die Einstecköffnung (22) des innenliegenden Stabteils (16) ein- oder aussteckbar ist.

5. Handhabungsinstrument (10) nach einem oder mehreren der vorstehenden Ansprüche, **dadurch gekennzeichnet, dass** das zweite Hebelelement (74) mindestens eine Aufnahmeöffnung (78) für das Kopplungselement (18) aufweist.

6. Handhabungsinstrument (10) nach einem oder mehreren der vorstehenden Ansprüche, **dadurch gekennzeichnet, dass** das erste oder zweite Hebelelement (70, 74) in einer Seitenansicht trapez-, dreieck- oder bogenförmig, insbesondere C-bogenförmig, angeordnete Streben (84, 86) aufweist, die einenends starr miteinander verbunden sind und anderenends nicht oder jedenfalls nicht starr miteinander verbunden sind.

7. Handhabungsinstrument (10) nach einem der vorhergehenden Ansprüche, **dadurch gekennzeichnet, dass** das außenliegende Gehäuseteil (14) in einem distalen Endbereich (40) zwei ungefähr halbschalenförmige in der Längsrichtung (12) diametral zueinander angeordnete Abschnitte (36) aufweist, die durch einen dazwischen ausgebildeten, in der Längsrichtung (12) erstreckten Spalt (38) begrenzt sind, und geringfügig quer zur Längsrichtung (12) gegeneinander auslenkbar sind, so dass sie in eine Hintergriffstellung am Kopf (2) der Knochenschraube (4) gelangen, insbesondere einrasten oder einschnappen, können und dabei zumindest einen Teil des Kopfs (2) der Knochenschraube (4) zwischen sich aufnehmen.

8. Handhabungsinstrument (10) nach einem der vorhergehenden Ansprüche, **dadurch gekennzeichnet, dass** durch Eingreifen des Stabteils (16) in die Werkzeugansetzstelle an der Knochenschraube (4) und durch einen Hintergriff in Umfangsrichtung (28) oder in axialer Längsrichtung (12) des außenliegenden Gehäuseteils (14) am Kopf (2) der Knochenschraube (4) eine Verspannung des Gehäuseteils (14) und des Stabteils (16) gegeneinander und gegenüber der Knochenschraube (4) bewirkbar ist.

9. Handhabungsinstrument (10) nach Anspruch 7, **dadurch gekennzeichnet, dass** ein Verriegelungselement (48) vorgesehen ist, das die halbschalenförmigen Abschnitte (36) des außenliegenden Gehäuseteils (14) zumindest teilweise derart übergreift, dass ein Aufspreizen der halbschalenförmigen Abschnitte (36) zumindest teilweise verhindert wird.

10. Handhabungsinstrument (10) nach Anspruch 9, **dadurch gekennzeichnet, dass** das insbesondere ringförmige Verriegelungselement (48) an dem mindestens einen Querstift (42) des Stabsteils (14) gehalten ist.

11. Handhabungsinstrument (10) nach einem der vorhergehenden Ansprüche, **dadurch gekennzeichnet, dass** in dem außenliegenden Gehäuseteil (14) wenigstens eine radiale Ausnehmung (52) ausgebildet ist, durch welche hindurch eine Markierung (50) an einer Außenseite des innenliegenden Stabteils (16) visuell wahrnehmbar ist, wenn sich das innenliegende Stabteil (16) in einer bestimmten Position in Längsrichtung (12) relativ zu dem außenliegenden Gehäuseteil (14) befindet.

12. Handhabungsinstrument (10) nach einem der vorhergehenden Ansprüche, **dadurch gekennzeichnet, dass** das innenliegenden Stabteil (16) und vorzugsweise das Kopplungselement (18) in axialer Längsrichtung (12) kanüliert ausgebildet sind, sodass ein Führungsdraht darin aufgenommen werden kann.

13. Handhabungsinstrument (10) nach einem der vorhergehenden Ansprüche, **dadurch gekennzeichnet, dass** das außenliegende Gehäuseteil (14) am proximalen Ende eine oder mehrere Werkzeugansetzstellen aufweist und ein passendes Werkzeug so angesetzt werden kann, dass damit das Handhabungsinstrument (10) um die axiale Längsrichtung (12) gedreht werden kann, um den Gewindeschaft (6) der Knochenschraube (4) in einen Knochen ein- bzw. auszuschrauben.

## Claims

1. Handling instrument (10) for a bone screw (4), in particular a pedicle screw, the bone screw (4) comprising a threaded shaft (6) and a head (2), in particular a fork head, and it being possible to connect the handling instrument (10) to the bone screw (4) in a releasable yet rigid and rotationally fixed manner, such that the threaded shaft (6) of the bone screw (4) can be screwed into a bone by means of the handling instrument (10), said handling instrument comprising an outer housing part (14) that extends in an axial longitudinal direction (12), is designed at least in portions in the shape of a tube or sleeve and comprises a wall, and said handling instrument comprising an inner, in particular cannulated, rod part (16) that is arranged in the outer housing part (14), the inner rod part (16) being able to engage in a torque-absorbing tool placement point of the bone screw (4), wherein the outer housing part (14) comprises at least one guide slot (24) that extends in the axial longitudinal direction (12) and that passes through a wall of the housing part, and wherein the inner rod part (16), in the region of the guide slot (24) of the outer housing part (14), comprises a transverse opening (22) that extends in particular in the radial direction, and wherein a coupling element (18) can be arranged in said transverse opening (22) of the inner rod part (16) arranged in the outer housing part (14), the coupling element (18) projecting into the guide slot (24) of the outer housing part (14) or projecting outwards through the guide slot (24) and in both cases being slidable in the longitudinal direction (12) in the guide slot (24) such that the inner rod part (16) and the outer housing part (14) are arranged so as to be shiftable in the axial longitudinal direction (12) relative to one another, but in a rotationally fixed manner on account of the coupling element (18), and wherein the rod part (16) can be loaded in the axial longitudinal direction (12) by means of the coupling element (18), **characterized in that** a toggle lever mechanism (68) is provided on the outer housing part (14), the toggle lever mechanism (68) comprising a first and a second lever element (70, 74) and the first lever element (70) being connected in an articulated manner to the second lever element (74) via a toggle joint (76) and **in that** the first lever element (70) being pivotally supported on the outer housing part (14) and the second lever element (74) being supported on the coupling element (18), and **in that** the toggle joint mechanism (68) being movable from a bent state into a stretched state and vice versa by actuating an operating element (80), such that by actuating the toggle lever mechanism (68), the coupling element (18) can be moved in the longitudinal direction (12) distally or proximally relative to the outer housing part (14) and **in that** the toggle lever mechanism (68) comprises a resilient element (82) which is formed by the first or second lever element (70, 74).

2. Handling instrument (10) according to claim 1, **characterized in that** the transverse opening (22) of the rod part (16) is an insertion opening into which the coupling element (18) can be inserted from the outside in the radial direction through the guide slot (24) of the outer housing part (14).

3. Handling instrument (10) according to claim 1 or 2, **characterized in that** the guide slot (24) has a slot width that varies in the longitudinal direction (12).

4. Handling instrument (10) according to claim 3, **characterized in that** the coupling element (18) tapers radially outwards, and the coupling element (18) can be inserted or removed through the guide slot (24) of the outer housing part (14) into the insertion opening (22) of the inner rod part (16) in at least one particular position, preferably the position having the greatest slot width.

5. Handling instrument (10) according to one or more of the preceding claims, **characterized in that** the second lever element (74) comprises at least one receiving opening (78) for the coupling element (18).

6. Handling instrument (10) according to one or more of the preceding claims, **characterized in that** the first or second lever element (70, 74) comprises braces (84, 86) that, in a side view, are arranged in the shape of a trapezium, triangle or arc, in particular in the shape of a C-arc, and that are rigidly interconnected at one end and at the other end are not interconnected or in any case not rigidly interconnected.

7. Handling instrument (10) according to any of the preceding claims, **characterized in that** the outer housing part (14), in a distal end region (40), comprises two approximately half-shell-shaped portions (36) that are arranged in the longitudinal direction (12) so as to be diametrically opposite one another, that are delimited by a gap (38) formed there between extending in the longitudinal direction (12), and that can be deflected away from one another slightly transversely to the longitudinal direction (12), such that they can enter a rear engagement position on the head (2) of the bone screw (4), in particular such that they can latch or snap in, and in the process receive between one another at least part of the head (2) of the bone screw (4).

8. Handling instrument (10) according to any of the preceding claims, **characterized in that**, by means of the rod part (16) engaging in the tool placement point on the bone screw (4) and by means of a rear engagement in the circumferential direction (28) or in the axial longitudinal direction (12) of the outer housing part (14) on the head (2) of the bone screw (4), the housing part (14) and the rod part (16) can be braced with respect to one another and with respect to the bone screw (4).

9. Handling instrument (10) according to any of the preceding claims, **characterized in that** a locking element (48) is provided which engages over the half-shell-shaped portions (36) of the outer housing part (14) at least in part such that the half-shell-shaped portions (36) are prevented from spreading apart at least in part.

10. Handling instrument (10) according to claim 9, **characterized in that** the in particular annular locking element (48) is held at the at least one transverse pin (42) of the rod part (14).

11. Handling instrument (10) according to any of the preceding claims, **characterized in that** at least one radial cut-out (52) is formed in the outer housing part (14), through which cut-out a marking (50) on an outer face of the inner rod part (16) can be visually perceived if the inner rod part (16) is in a particular position relative to the outer housing part (14) in the longitudinal direction (12).

12. Handling instrument (10) according to any of the preceding claims, **characterized in that** the inner rod part (16) and preferably the coupling element (18) are cannulated in the axial longitudinal direction (12), such that a guide wire can be received therein.

13. Handling instrument (10) according to any of the preceding claims, **characterized in that** the outer housing part (14) comprises one or more tool placement points at the proximal end and a suitable tool can be attached such that the handling instrument (10) can be rotated about the axial longitudinal direction (12) in order to screw the threaded shaft (6) of the bone screw (4) into or out of a bone.

## Revendications

1. Instrument de manipulation (10) pour une vis à os (4), en particulier une vis pédiculaire, dans lequel ladite vis à os (4) présente une tige filetée (6) et une tête (2), en particulier une tête fourchue, et l'instrument de manipulation (10) peut être relié de manière amovible, mais rigide et solidaire en rotation à la vis à os (4) afin de pouvoir visser la tige filetée (6) de la vis à os (4) dans un os au moyen de l'instrument de manipulation (10), avec une partie de boîtier (14) située à l'extérieur qui s'étend dans une direction longitudinale axiale (12), est réalisée - au moins par sections - en forme de tube ou de manchon et présente une paroi ainsi qu'avec une partie de tige (16) située a l'intérieur, en particulier canulée, qui est disposée à l'intérieur de la partie de boîtier (14) située à l'extérieur, dans lequel ladite partie de tige (16) située à l'intérieur peut s'engager dans un point d'application d'outil de la vis à os (4), qui absorbe le couple, dans lequel la partie de boîtier (14) située à l'extérieur présente au moins une fente de guidage (24) s'étendant dans la direction longitudinale axiale (12) et traversant une paroi de la partie de boîtier et la partie de tige (16) située à l'intérieur présente, au niveau de la fente de guidage (24) de la partie de boîtier (14) située à l'extérieur, une ouverture transversale (22) s'étendant en particulier dans la direction radiale, et dans lequel un élément de couplage (18) peut être disposé dans cette ouverture transversale (22) de la partie de tige (16) située à l'intérieur qui est agencée dans la partie de boîtier (14) située à l'extérieur, dans lequel ledit élément de couplage (18) fait saillie dans la fente de guidage (24) de la partie de boîtier (14) située à l'extérieur ou fait saillie vers l'extérieur à travers la fente de guidage (24) et, dans les deux cas, peut être déplacé à glissement suivant la direction longitudinale (12) dans la fente de guidage (24) de sorte que la partie de tige (16) située à l'intérieur et la partie de boîtier (14) située à l'extérieur peuvent être déplacées l'une par rapport à l'autre dans la direction longitudinale axiale (12) mais sont disposées de manière solidaire en rotation au moyen de l'élément de couplage (18), et dans lequel la partie de tige (16) peut être chargée dans la direction longitudinale axiale (12) au moyen de l'élément de couplage (18), **caractérisé par le fait qu'**un mécanisme à genouillère (68) est prévu sur la partie de boîtier (14) située à l'extérieur, dans lequel ledit mécanisme à genouillère (68) comprend des premier et deuxième éléments de levier (70, 74) et le premier élément de levier (70) est relié de manière articulée par une articulation de genou (76) au deuxième élément de levier (74), et que le premier élément de levier (70) est appuyé à pivotement sur la partie de boîtier (14) située à l'extérieur et le deuxième élément de levier (74) est appuyé sur l'élément de couplage (18), et le mécanisme à genouillère (68) peut être déplacé, en actionnant un élément de commande (80), d'un état fléchi à un état étiré et vice versa, de sorte qu'en actionnant le mécanisme à genouillère (68), l'élément de couplage (18) peut être déplacé dans la direction longitudinale (12) de manière distale ou bien proximale par rapport à la partie de boîtier (14) située à l'extérieur, et que le mécanisme à genouillère (68) comprend un élément élastique (82) qui est formé par le premier ou le deuxième élément de levier (70, 74).

2. Instrument de manipulation (10) selon la revendication 1, **caractérisé par le fait que** l'ouverture transversale (22) de la partie de tige (16) est une ouverture d'engagement dans laquelle l'élément de couplage (18) peut être engagé de l'extérieur dans la direction radiale à travers la fente de guidage (24) de la partie de boîtier (14) située à l'extérieur.

3. Instrument de manipulation (10) selon la revendication 1 ou 2, **caractérisé par le fait que** la fente de guidage (24) présente une largeur de fente qui varie dans la direction longitudinale (12).

4. Instrument de manipulation (10) selon la revendication 3, **caractérisé par le fait que** l'élément de couplage (18) est rétréci radialement vers l'extérieur, et l'élément de couplage (18) peut être engagé dans ou sorti de l'ouverture d'engagement (22) de la partie de tige (16) située à l'intérieur par la fente de guidage (24) de la partie de boîtier (14) située à l'extérieur, sur au moins une position déterminée, de préférence celle présentant la plus grande largeur de fente.

5. Instrument de manipulation (10) selon l'une ou plusieurs des revendications précédentes, **caractérisé par le fait que** le deuxième élément de levier (74) présente au moins une ouverture de réception (78) pour l'élément de couplage (18).

6. Instrument de manipulation (10) selon l'une ou plusieurs des revendications précédentes, **caractérisé par le fait que** le premier ou le deuxième élément de levier (70, 74) présente, dans une vue de côté, des entretoises (84, 86) qui sont agencées en forme de trapèze, de triangle ou d'arc, en particulier en forme d'arc de C, et qui sont reliées rigidement entre elles à une extrémité et ne sont pas reliées entre elles ou en tout cas ne sont pas reliées rigidement entre elles à l'autre extrémité.

7. Instrument de manipulation (10) selon l'une quelconque des revendications précédentes, **caractérisé par le fait que** la partie de boîtier (14) située à l'extérieur présente, dans une zone d'extrémité distale (40), deux sections (36) à peu près en forme de demi-coque qui sont disposées diamétralement l'une par rapport à l'autre dans la direction longitudinale (12) et qui sont délimitées par une fente (38) réalisée entre elles et s'étendant dans la direction longitudinale (12), et qui peuvent être déviées légèrement l'une par rapport à l'autre transversalement à la direction longitudinale (12) de sorte qu'elles peuvent arriver dans une position dans laquelle elles se prennent derrière la tête (2) de la vis à os, en particulier par encliquetage ou enclenchement, tout en recevant ainsi entre elles au moins une partie de la tête (2) de la vis à os (4).

8. Instrument de manipulation (10) selon l'une quelconque des revendications précédentes, **caractérisé par le fait que** l'on peut provoquer un serrage de la partie de boîtier (14) et de la partie de tige (16) l'une contre l'autre ainsi que par rapport à la vis à os (4) par un engagement de la partie de tige (16) dans le point d'application d'outil sur la vis à os (4) et par une prise de derrière dans la direction circonférentielle (28) ou dans la direction longitudinale axiale (12) de la partie de boîtier (14) située à l'extérieur sur la tête (2) de la vis à os (4).

9. Instrument de manipulation (10) selon la revendication 7, **caractérisé par le fait qu'**un élément de verrouillage (48) est prévu qui recouvre au moins en partie lesdites sections en forme de demi-coque (36) de la partie de boîtier (14) située à l'extérieur de telle manière qu'un écartement des sections en forme de demi-coque (36) est empêché au moins en partie.

10. Instrument de manipulation (10) selon la revendication 9, **caractérisé par le fait que** l'élément de verrouillage (48) en particulier annulaire est maintenu sur ladite au moins une broche transversale (42) de la partie de tige (14).

11. Instrument de manipulation (10) selon l'une quelconque des revendications précédentes, **caractérisé par le fait que** dans la partie de boîtier (14) située à l'extérieur est réalisé au moins un évidement radial (52) à travers lequel un marquage (50) se trouvant sur une face extérieure de la partie de tige (16) située à l'intérieur est visuellement perceptible lorsque la partie de tige (16) située à l'intérieur se trouve dans une position déterminée dans la direction longitudinale (12) par rapport à la partie de boîtier (14) située à l'extérieur.

12. Instrument de manipulation (10) selon l'une quelconque des revendications précédentes, **caractérisé par le fait que** la partie de tige (16) située à l'intérieur et de préférence l'élément de couplage (18) sont réalisés de manière à être canulés dans la direction longitudinale axiale (12) de sorte qu'un fil de guidage peut être logé à l'intérieur de ceux-ci.

13. Instrument de manipulation (10) selon l'une quelconque des revendications précédentes, **caractérisé par le fait que** la partie de boîtier (14) située à l'extérieur présente un ou plusieurs points d'application d'outil à l'extrémité proximale et qu'un outil approprié peut être appliqué de telle manière que l'instrument de manipulation (10) peut être tourné par celui-ci autour de la direction longitudinale axiale (12) afin de visser la tige filetée (6) de la vis à os (4) dans un os ou bien de la dévisser de celui-ci.
